Europäisches Patentamt

European Patent Office

Office européen des brevets

(19)

(11) Publication number: **0 062 924**

**A2**

(12) # EUROPEAN PATENT APPLICATION

(21) Application number: **82103147.3**

(22) Date of filing: **14.04.82**

(51) Int. Cl.³: **A 61 K 39/015**

(30) Priority: **15.04.81 GB 8111875**
**03.08.81 GB 8123710**

(43) Date of publication of application:
**20.10.82 Bulletin 82/42**

(84) Designated Contracting States:
**BE CH DE FR GB LI NL SE**

(71) Applicant: **THE WELLCOME FOUNDATION LIMITED**
**183-193 Euston Road**
**London NW1 2BP(GB)**

(72) Inventor: **Holder, Anthony Arthur**
**8 Richmond Close**
**Biggin Hill Kent(GB)**

(72) Inventor: **Freeman, Robert Rowe**
**23 Bennett Park**
**Blackheath London, S.E.3(GB)**

(74) Representative: **Berg, Wilhelm, Dr. et al,**
**Dr. Berg, Dipl.-Ing. Stapf, Dipl.-Ing. Schwabe, Dr. Dr.**
**Sandmair Mauerkircherstrasse 45**
**D-8000 München 80(DE)**

(54) Protozoal antigen.

(57) Protection inducing antigens of parasites of the genus *Plasmodium* is described. The antigens have an apparent molecular weight of 2.0 to 2.6 × 10⁵ and are associated with a localised region of the erythrocytic merozoite forms of the parasite. The antigens may be incorporated into vaccines and used for the inducing of immunity into susceptible vertebrate hosts including humans. Methods for the preparation of the antigens are also described.

EP 0 062 924 A2

Croydon Printing Company Ltd.

A642F
0062924

The Wellcome Foundation Limited


PROTOZOAL ANTIGEN


The present invention relates to antigenic material, to vaccines containing it and to the use thereof to provide immunity to malaria.

The malaria parasites are protozoa belonging to the genus Plasmodium. Part of their complex life cycle involves cyclic asexual replication within the erythrocytes of the vertebrate host, giving rise to the symptoms of the disease known as malaria. The vertebrate host may, in turn, exhibit a protective immune response which is effective against the blood-borne parasites. It is implied, therefore, that the blood forms synthesize antigens which elicit the production of specific, protective antibodies in the infected or immunized host.

During the asexual erythrocytic cycle of replication, free parasites (merozoites) recognize and attach specifically to the surface of erythrocytes, and then invade them by invagination of the plasma membrane. The infected erthrocyte is known as the trophozoite. During the intracellular differentiation of the parasite the nucleus undergoes repeated divisions, to form the schizont. Nuclear division is followed by segmentation of the cytoplasm to form a number of uninucleate intraerythrocytic merozoites. Upon lysis of the host erythrocyte membranes, the merozoites are released into the plasma and may attach to, and invade, fresh host erythrocytes.

It has long been suspected that the protection-inducing antigen(s) might be situated on the surface of the free merozoites, since these are the only forms exposed directly to the antibody-containing plasma. This was implied by the results of Cohen, Butcher and Crandall (Nature, 1969, 223 368-371), who showed inhibition of the cycle of erythrocytic replication of Plasmodium knowlesi in vitro at the point where merozoites were released into the medium containing specific antibody. These workers speculated that the action of antibody in this system was to block specific merozoite surface antigens involved in attachment to host erythrocyctes. Subsequently, Mitchell, Butcher and Cohen (Immunology, 1975, 29 397-407), used purified merozoites emulsified in Freund's Complete Adjuvant to successfully vaccinate rhesus monkeys against P. knowlesi malaria.

CLB/JAH/18th March,1982.

There is no reason to expect that the mechanism of protective immunity against malaria parasites of rodents should differ substantially from that operating against malaria parasites of primates. Thus, in addition to those found for primate parasites, protective antibodies against the rodent malaria parasites have also been identified. For example antibody against P. yoelii appears to act against late schizonts and/or merozoites (Freeman and Parish, Experimental Parasitology, 52;18-24, 1981) and two merozoite-specific monoclonal antibodies were protective when injected into P. yoelii-infected mice (Freeman, Trejdosieicz and Cross, Nature, 1980, 284: 366-368, 1980. Freeman, Holder, Tredjdosiewicz and Cross, in "The Host-Invader Interplay" (H. Van den Bossche, ed.) Elsevier/North Holland, 1980, pp. 121-124).

Attempts have been made to define the diversity of protein antigens associated with merozoites (see for example Deans et al, Parisitology 1978, 77, 333-344; Miller et. al, J. exp. Med, 1980, 151, 750-798 and Kilejian, Proc. Nat. Acad. Sci, 1980, 77, 3695-3699). However no specific antigens capable of inducing a protective response by the host or specifically recognised by such a protective response have been isolated and characterised.

There has now been isolated and defined such a series of antigens associated with merozoite forms of these Plasmodium parasites.

The invention accordingly provides, in a first aspect, a protection-inducing proteinaceous antigen of parasites of the genus Plasmodium having the following characteristics:-

(i) a molecular weight in the range of $2.0 \times 10^5$ to $2.6 \times 10^5$; and
(ii) associated with localised region of the erythrocytic merozoite forms of the parasites;
and functional derivatives thereof.

By "molecular weight" is meant the apparent molecular weight as determined by polyacrylamide gel electrophoresis and standard molecular weight markers. The molecular weight of the antigenic proteins of the invention may thus be conveniently determined by the techniques described by U.K. Laemmli, in Nature, 1970, 227, 680-685. Convenient standard molecular weight markers include, for example, spectrin heterodimer ($2.2 \times 10^5$ molecular weight and $2.4 \times 10^5$ molecular weight).

CLB/JAH/18th March,1982.

Antigenic proteins of the present invention having a molecular weight of about $2.35 \times 10^5$ have been found to be particularly advantageous.

The antigenic proteins of the present invention are known to occur in a number of murine malaria protozoa such as Plasmodium yoelii, P. berghei, P. vinckei petteri and P. chabaudi, and are also to be found the malaria parasites of primates in particular the human parasite P.falciparum. The term 'associated' as used herein refers not only to proteinaceous antigenic material originating from the merozoite forms of malaria parasites but to antigenically identical material of similar or identical amino acid sequence deriving from any other source.

The antigens of the present invention are, in the malaria parasite, to be found only in the later stages of the parasite growth cycle, that is to say predominantly in the intraerythrocyctic development when individual merozoites are being formed. The antigens of the present invention are not found during the ring and trophozoite stages. Thus, for P.falciparum, which has a development cycle of about 48 hours, no antigen is found during the first 30 hours of development, but maximal synthesis occurs during the period of about 36 to 45 hours which is late compared with the peak of total protein synthesis occuring at about 36 hours.

As will be appreciated by those skilled in the art any material which possesses the antigenic properties of the proteinaceous material defined above including fragments thereof and material incorporating the antigen or fragments thereof will have the same benefical immunogenic properties as the parent antigen. Such materials are referred to herein as "functional derivatives".

The antigenic proteins of the present invention may be prepared by any method known in the art for the preparation of such antigens. All such methods comprise either isolation of the antigenic protein from the parasite or chemically or biologically reproducing the antigenic protein, for example full or partial chemical synthesis or expression from suitably genetically modified hosts by the methods of genetic manipulation.

The antigenic proteins of the invention may be isolated from the merozoite form of the parasite by means of monoclonal antibodies specific for the antigens of the invention. The technique of antigen separation by means of monoclonal antibodies has not previously been applied to the purification of malaria antigens.

The invention thus provides in a further aspect a method for the preparation of an antigenic protein as defined herein which comprises the steps of:-

(i)     solubilising erythrocytes containing the merozoite form of a Plasmodium parasite;

(ii)    contacting the solubilised material with a monoclonal antibody specific for the desired antigenic protein to provide an antibody antigen complex; and

(iii)recovering the antigenic protein from the antibody - antigen complex.

The invention also provides an antigenic protein as defined herein when prepared by the above defined method. The merozoites may be solubilised by any method known in the art for effecting such solubilisation. Suitably conditions which solubilise parasite material without extensive proteolysis and denaturation are employed. In particular they may be solubilised by contacting them with a detergent, which may be of the ionic or non-ionic type although non-ionic detergents are preferred. Examples of such detergents include Nonidet P40, Triton X-100, Brij 99 (Registered Trade Marks, manufactured respectively by Shell, Rohm and Haas and ICI) and a polyoxyethylene (12) tridecyl ether detergent as known Renex 30 (Registered Trade Mark, manufactured by Honeywell Atlas Limited). Detergent is added to a final concentration of between 0.01% and 5% v/v.

Monoclonal antibodies for use in the method maybe prepared by any method known in the art for their production (see for example Milstein, Scientific American 1980, 243 (4), 56-64). In such a method a mouse is immunised against the parasite concerned, in this case a malaria parasite for example by infection with a rodent malaria parasite. Lymphocytes each of which will have the independent capacity to make an antibody that recognises a different antigenic determinant, are then isolated and fused with mouse myeloma cells to provide a "hybridoma". Each hybridoma will be capable of expressing the antibody of its parent lymphocyte and of being cloned. Screening of individual hybridomas provides one or more cell lines expressing the antibody, a monoclonal antibody, specific for the antigenic determinant of interest and the cell-line then used to generate large quantities

CLB/JAH/18th March,1982.

of the monoclonal antibody which may then be used to separate and purify the antigenic determinant of interest. The identification of antibodies specific for the antigens of the invention may be readily determined by simple tests well known in the art. A cell-line, the monoclonal antibody derived therefrom and attempts to identify the antigens from Plasmodium yoelii recognised by the antibody have been referred to in the literature (Freeman et. al. The Host Invader Interplay, H. Van den Bosche Ed., Elsevier/North Holland Biomedical Press 1980, 121-124) but no specific antigens were isolated and only partial characterisation of the monoclonal antibody and cell line used to isolate the antigens given.

The monoclonal antibody is suitably bonded to an inert support material, for example Sepharose, and the solubilised material passed through the support containing antibody. This may conveniently be effected by means of a column.

The antigenic protein may be recovered from the antibody-antigen complex by disruption of the complex. The conditions for effecting this are well known in the art. Suitable conditions which preserve the immunogenicity of the protein include washing with thiocyanate ions, for example potassium thiocyanate, in the presence of a suitable detergent.

The antigen described above may be incorporated into a vaccine for inducing immunity to Malaria in susceptible veterbrate hosts at risk of becoming infected by parasites of the genus Plasmodium. For this purpose the antigenic protein may be presented in association with a pharmaceutically acceptable carrier.

According to the present invention in a further aspect there is provided a vaccine for inducing immunity to Malaria which comprises an antigen as hereinbefore defined in association with a pharmaceutically acceptable carrier.

Pharmaceutically acceptable carriers, in this instance, are liquid media suitable for use as vehicles to introduce the antigen into the patient. An example of such a carrier is saline solution. The antigenic protein may be in solution or suspended as a solid in the carrier, or it may be solubilised by the addition of pharmaceutically acceptable detergent.

CLB/JAH/18th March,1982.

The vaccine may also comprise an adjuvant for stimulating the immune response and thereby enhancing the effect of the vaccine. Convenient adjuvants for use in the present invention include Freunds complete adjuvant and more particularly, saponin, Corynebacterium parvum (coparvax) and aluminium hydroxide or a mixture of these or other known adjuvants.

Conveniently the vaccines are formulated to contain a final concentration of antigenic protein in the range of from 0.2 to 5 mg/ml, preferably 0.5 to 2 mg/ml, most preferably 1 mg/ml. After formulation the vaccine may be incorporated into a sterile container which is then sealed and stored at a low temperature, for example $4^{o}C$, or may be freeze dried.

In order to induce immunity in veterbrate hosts to malaria one or more doses of the vaccine suitably formulated may be administered. It is recommended that each dose is 0.1 to 2 ml preferably 0.2 to 1 ml, most preferably 0.5 ml of vaccine.          The present invention in a further aspect provides a method for inducing immunity to malaria in susceptible veterbrate hosts, comprising the administration of an effective amount of a vaccine, as hereinbefore defined, to the host.

The vaccines of the present invention may be administered by any conventional method for the administration of vaccines including oral and parenteral (eg. subcutaneous or intramuscular) injection. The treatment may consist of a single dose of vaccine or a plurality of doses over a period of time.

The following Examples serve to illustrate the invention but are not intended to limit it in any way.

EXAMPLE 1 Derivation of Hybridoma Line

Spleen cells from two P. yoelii- immune BALB/c mice were fused with P3-NS1/1-Ag4-1 myeloma cells in the presence of polyethylene glycol (by the general method of Galfre, et al., Nature 1977, 266, 550-552). The cells were dispensed into 144 tissue culture wells in 2 ml volumes of HAT selective medium (Littlefield, Science 1964, 145, 709-710) using RPMI 1640 medium supplemented with 10% foetal calf serum as a base. After 10 days the culture supernatants were tested for P.yoelii-specific

CLB/JAH/18th March,1982.

antibody by indirect immunofluorescence (IIF). Of 143 cultures tested, 38 were positive. Culture number 77 contained an antibody specific for an antigen associated with merozoites but absent from ring-forms and tropho-zoites. The antibody binding did not appear to be uniform but was localised to a region on or within each merozoite. The antibody did not appear to react with free intact merozoites in solution, suggesting that the localisation was within the parasite. A cloned hybridoma cell line was produced from this culture by growing colonies derived from single cells in semi-solid agar overlayed with medium. The cloned hybridoma line continued to secrete its unique antibody, and was designated W1C 25.77. Cell line WIC 25.77 has been deposited at the Institute Pasteur under the number I- 161 on 16 July 1981. The hybridoma line was further grown as an ascites tumor in BALB/c mice, and ascitic fluid and serum from these mice contained about 10 mg/ml of the monospecific antibody. Two other hybridoma lines, WIC25.37 and WIC 25.86 secrete antibodies which are specific for the same antigen as is recognised by antibody secreted by cell line WIC 25.77.

EXAMPLE 2     Preparation of Antigen associated with P. yoelii

Blood forms Plasmodium yoelii were grown in CD-1 mice to 80-90% parasitaemia and the erthrocytes were harvested and solubilised. The cells were solubilised by lysis at $4^{o}$C in a buffer of 50mM Tris, 5mM ethylenediamine tetraacetate (EDTA), 1% Nonidet P40 pH 8.0 containing 1mM phenylmethyl sulphonyl fluoride (PMSF), 0.1mM tosyl-L-lysine chloromethyl ketone (TLCK), 5mM ethyleneglycol-bis-(amino ethyl ether)N,N'-tetraacetate (EGTA) and 5mM iodoacetamide to inhibit proteolytic activity. The lysed cells were centrifuged at 100,000 g and the supernatant, containing the erythrocyte soluble proteins and some membrane proteins, together with approximately 70% of the parasite proteins (as estimated from the proportion of $^{35}$S-methionine label in this fraction after an in vitro incorporation) collected.

Immunoglobulin from the ascites fluid of mice containing hybridoma line W1C 25.77 was purified by binding to Protein A- Sepharose and a single step elution at pH 3.0. The immunoglobulin fraction was coupled to cyanogen bromide activated Sepharose, using 10 mg immunoglobulin/ml swollen gel. The immunoabsorbant was extensively washed, packed into columns and equilibrated with 10mM Tris, 1mM EDTA, 1mM EGTA, 1% NP40 pH 8.0.

CLB/JAH/18th March,1982.

The supernatant obtained as described above was passed through the immunoabsorbant equilibrated with 10mM Tris, 1mM EDTA, 1mM EGTA, 1% NP40 pH 8.0 and washed through with this buffer. Non specifically bound material was removed by washing (5 column volumes) with this buffer containing 0.5M NaCl. The column was then washed with a buffer containing 10mM Tris, 1mM EDTA, 1mM EGTA, 0.01% NP40, pH 8.0. Specific elution was achieved using the same buffer containing 2M potassium thiocyanate.

The eluted material was subjected to a second cycle of immunoabsorbant chromatography. The specific eluate was concentrated to 10% of its volume and then dialysed against a buffer containing 10mM Tris, 1mM EDTA, 1mM EGTA, 0.1% NP40 pH 8.0. The sample was applied to the column which had been re-equilibrated in this buffer and then the retained material was eluted with 10mM Tris, 1mM EDTA, 1mM EGTA, 0.01% NP40 and 2M KSCN pH 8.0. The eluate was concentrated and dialysed to provide the antigen.

EXAMPLE 3    Biochemical Characterization of the Antigen associated with P. yoelii

The antigenic material prepared in Example 2 was subjected to SDS gel electrophoresis and the single protein band had an estimated molecular weight of $2.35 \times 10^5$ relative to the molecular weight markers of the spectrin heterodimer ($2.2 \times 10^5$ and $2.4 \times 10^5$ molecular weights) using a 7.5% polyacrylamide gel.

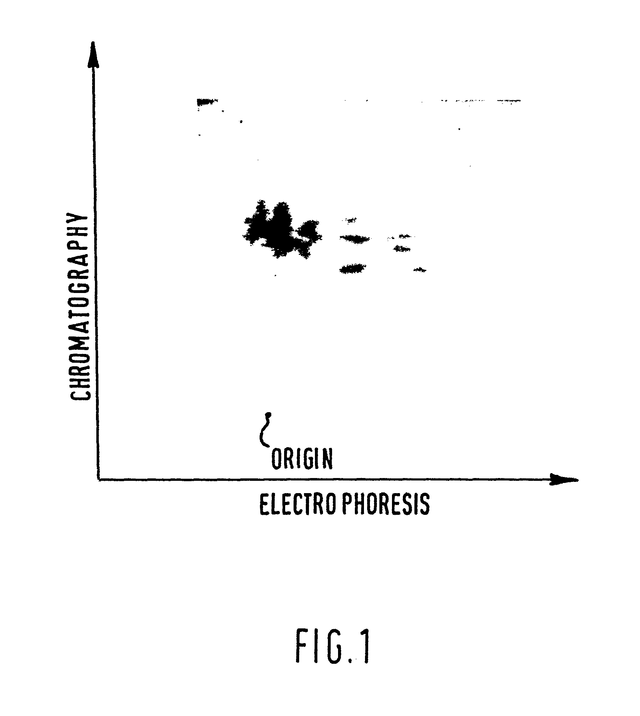

$^{35}$S-methionine was incorporated into the protein by incubating parasitized cells in a medium containing this amino acid at a high specific activity. The labelled antigenic protein was obtained by immunoprecipitation with the monoclonal antibody 25.77 and Protein A bearing Staphylococcus aureus cells to precipitate the immune complex. When this material was subjected to SDS gel electrophoresis a single species of $2.35 \times 10^5$ apparent molecular weight was detected by autoradiography. When a gel slice containing the antigenic protein was incubated in the presence of chymotrypsin, peptide digestion products were released. These peptides were mapped by thin layer electrophoresis at pH 4.4 followed, at right angles, by chromatography in butanol-acetic acid-water-pyridine. The chymotryptic map of $^{35}$S-methionine containing peptides so obtained is shown in Figure 1. Standard tests including periodic acid – Schiffs staining or $^{125}$I-iodinated Concavalin A lectin binding to the protein in acrylamide gels indicated that the protein was not glycosylated. The lack of glycoslation was confirmed by inability to specifically bind and elute protein from lentil lectin-sepharose and wheat germ agglutinin-sepharose and by the absence of any effect on the size of the protein when synthesised by the parasite in the presence of tunicamycin, a specific inhibitor of protein N-glycosylation.

The isoelectric point of the protein was determined using a two-dimensional gel electrophoresis system based in that described by O'Farrell (J. Biol. Chem. 250 : 4007-4021, 1975) but using an agarose polyacrylamide gel in the first dimension. When the pH gradient was generated using pH3-10 ampholines, the protein had an isoelectric point within the range pH6 ± one pH unit.

EXAMPLE 4    Immunisation of mice with an antigen of Plasmodium yoelii, purified using monoclonal antibody 25.77: antibody response

Groups of 5 BALB/c mice were immunised with the antigenic protein obtained from Example 2 or with control preparations according to the following schedule:-

Group 1 :    20 µg antigen 25.77 emulsified in Freund's Complete Adjuvant (FCA) and injected intraperitioneally (i.p.) in a volume of 0.2 ml on day 0.

20 µg antigen 25.77 suspended in 0.2 mls normal mouse serum and injected intravenously on day 18.

20 µg antigen 25.77 suspended in saline and injected i.v. on day 42.

Group 2 :    FCA only, given i.p. on day 0
NMS only, given i.v. on day 18
Saline only, given i.v. on day 42.

On day 49, serum samples were taken for titration and examination by indirect immunofluorescence (IIF). The results are shown below.

CLB/JAH/18th March,1982.

| Group specificity | Mouse | IIF titre | Antiserum |
|---|---|---|---|
| 1 | 1 | > 1 : 1280 | merozoite |
|  | 2 | > 1 : 1280 | merozoite |
|  | 3 | > 1 : 1280 | merozoite |
|  | 4 | > 1 : 1280 | merozoite |
|  | 5 | > 1 : 1280 | merozoite |
| 2 | 1 | < 1 : 40 | - |
|  | 2 | < 1 : 40 | - |
|  | 3 | < 1 : 40 | - |
|  | 4 | < 1 : 40 | - |
|  | 5 | < 1 : 40 | - |

The pattern of fluorescent staining using the serum from the mice immunised with antigen 25.77 was indistinguishable from that produced using monoclonal antibody 25.77. Staining was restricted to the merozoite forms of P. yoelii. This result confirmed the purity and immunogenic nature of antigen 25.77 prepared as described in Example 2.

EXAMPLE 5      Immunisation of mice with an antigen of Plasmodium yoelii, purified using monoclonal antibody 25.77: protection against challenge infection

Groups of 5 BALB/c mice were immunised with the antigenic protein obtained from Example 2, or with control preparations according to the following schedule:-

Group 1:      12 μg antigen 25.77 emulsified in FCA and injected i.p. in a volume
of 0.2 mls on day 0.
12 μg antigen 25.77 in FCA i.p. on day 35.
20 μg antigen in 0.1 ml saline i.v. on day 50.

Group 2:      2 μg antigen 25.77 in FCA i.p. on day 0.
2 μg antigen 25.77 in FCA i.p. on day 35.
20 μg antigen 25.77 in 0.1 ml saline i.v. on day 50.

Group 3:          Saline in FCA i.p. on day 0.

                  Saline in FCA i.p. on day 35.

                  0.1 ml saline given i.v. on day 50.


On day 60, serum samples were taken for IIF titration of antibody, and for analysis by immunoprecipitation. On day 61 all mice were challenged i.v. with $10^4$ P.yoelii YM-parasitised erythrocytes. The antibody response of the groups on immunised mice are given in Table 1. The results demonstrate that under the conditions used, immunisation with 12 µg of antigen 25.77 was more effective in inducing an antibody response than was immunisation with 2 µg if antigen 25.77.

Table 1 :          Antibody response of mice immunised with antigen 25.77


| Group | IIF titre | antiserum specificity |
|---|---|---|
| 1 | $> 1:10, 240$ | merozoites |
| 2 | $> 1:640$ | merozoites |
| 3 | $< 1:40$ | nonspecific |


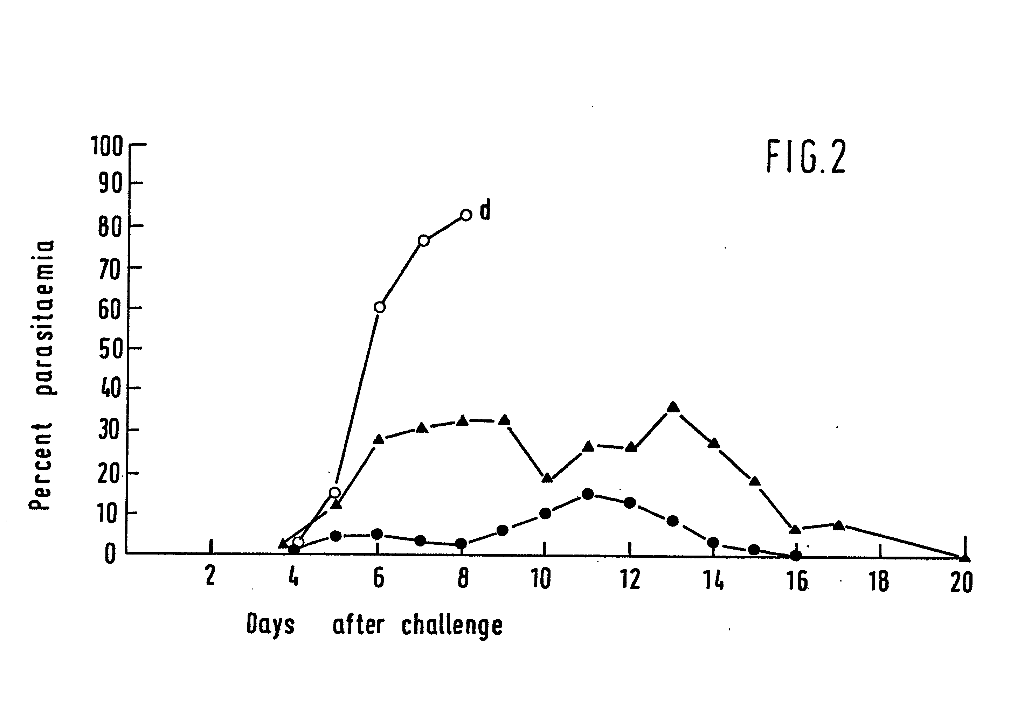

Figure 2: Mean parasitaemias in groups of 5 immunised or control mice after challenge with P.yoelii YM. Group 1 (•), Group 2 (▲), and Group 3 (○).


The protection against challenge with P.yoelii provided to each group shown in Figure 2. The results show that the group with the higher antibody titre were more effectively protected against challenge. The control group of mice all died within 8 days of challenge.


The antigenic specificity of serum from the immunised mice was checked by immunoprecipitation which demonstrated that the antibody response was directed against the 235,000 m.w. merozoite-specific antigen used for immunisation.


CLB/JAH/18th March,1982.

EXAMPLE 6    Derivation of merozoite-specific monoclonal antibodies
             specific for P. falciparum

BALB/c mice were immunised intraperitoneally on 2-4 occasions with
$10^8$ P. falciparum schizont - infected human red cells,  then were boosted
intravenously with a similar inoculum,  three days before fusion of spleen
cells with P3-NS1/1-Ag4-1 myeloma cells as described in Example 1.  Seven
cultures contained antibodies reacting with P. falciparum merozoites in
the IIF test,  and hybridoma lines from these cultures were cloned and grown
up in BALB/c mice as ascites tumors.  The hybridoma lines so obtained
all secrete antibodies which give IIF patterns on P. falciparum merozoites
similar to the IIF pattern of the antibody of Example 1 on P. yoelii merozoites.

Alternatively, BALB/C mice were immunised intraperitoneally with
100ug protein which had been purified from lysates of P.falciparum infected
cells by retention on an affinity column containing immunoglobulins from
13 pooled human immune sera.  Three weeks later the mice were boosted
intravenously with a similar innoculum, and fusion of spleen cells with P3-NSI/IAg4-1
cells was performed 3 days later.

EXAMPLE 7    Characterisation of high molecular weight protein antigens
             from P. falciparum and their recognition by human immune sera

A schizont enriched fraction of parasitised erythrocytes,  prepared
by centrifugation through a layer of Percoll was labelled with $^{35}$S-methionine
during a period of 2 hours.  The cells were harvested and solubilised by
lysis at $4^0$ as described in Example 2.  After centrifugation the supernatant
was analysed by SDS gel electrophoresis on a 5% polyacrylamide gel.  In
addition to many smaller polypeptides,  the material contained major labelled
polypeptides of 2.05,  2.18,  2,25,  2.35, 2.50 and 2.58 x $10^5$ apparent molecular
weights.

Sera from 14 immune Cambian donors were tested in vitro in a P.
falciparum invasion inhibition assay.  During a sinle cycle of invasion, these
sera inhibited invasion by 16-90%.  Ther sera were then tested for immunopre-
cipitation of $^{35}$S-methionine labelled polypeptides.  The immune sera precipitated at least 40 polypeptides, of which 10 were of molecular greater than
2.0 x $10^5$.  In particular a polypeptide of 2.35 x $10^5$ MW was preciptated

A642F

strongly by those sera which inhibit most effectively, but was precipitated only weakly by those sera which were least effective in the invasion inhibition assay.

When intraerthrocytic parasite development in vitro was synchronised by two treatments with sorbitol 33 hours apart the timing of polypeptide synthesis during one 48 hour cycle could be investigated. Schizonts were first observed 27 hours after the second sorbitol treatment and the first reinvasion occured at 39 hours. At six hour intervals the polypeptides synthesised during a 30 minute pulse with $^{35}$S-methionine were investigated. No 2.35 x $10^5$ MW polypeptide recognised by human immune system was synthesised at 6, 12, 18 and 24 hours after synchronisaction (ie during ring and trophozoite stages). A very small amount of synthesis of this polypeptide was detected at 30 hours, but the majority of synthesis was detected at 36, 42 and 45 hours. The peak of total protein synthesis during schizogeny occured at 36 hours, therefore this protein is synthesised late during the intraerthroytic development, when individual merozoites are formed.

EXAMPLE 8  Vaccines

Vaccines for use in immunisation may be prepared conventional techniques with the following constituents:-

Formulation A

| Antigen | 2 mg |
| Physiological Saline to | 1 ml |

Formulation B

| Antigen | 1 mg |
| Alhydrogel | 1 mg |
| physiological Saline to | 1 ml |

CLB/JAH/18th March, 1982.

CLAIMS

1.  A protection-inducing proteinaceous antigen of parasites of the genus
    <u>Plasmodium</u> characterised by:-

    (i) having an apparent molecular weight in the range of 2.0 to 2.6 x $10^5$;
    and

    (ii) being associated with a localised region of the erythrocytic merozoite
    forms of the parasite;
    and functional derivatives thereof.

2.  An antigen as claimed in claim 1 characterised in that the apparent
    molecular weight of the antigen is about 2.35 x $10^5$.

3.  An antigen as claimed in claim 1 or claim 2 characterised in that
    the parasite is a human <u>Plasmodium</u> parasite.

4.  An antigen as claimed in claim 3 charactersied in that the parasite
    is <u>Plasmodium falciparum</u>.

5.  An antigen as claimed in any one of claims 1 to 5 characterised in
    that it is prepared by the steps of:-

    (i) solubilising erythrocytes containing the merozoite form of a <u>Plasmodium</u>
    parasite;

    (ii) contacting the solubilised material with a monoclonal antibody
    specific for the desired antigen to provide an antibody-antigen complex;
    and

    (iii) recovering the antigen from the antibody-antigen complex.

6.  An antigen as claimed in any one of claims 1 to 4 characterised in
    that it is prepared by a chemical or biological reproduction.

CLB/JAH/18th March, 1982.

A642 EUR **0062924**

7. A mtheod for the preparation of an antigen as defined in any one of claims 1 to 4 which method comprises:-

   (a) isolating the antigen from the erythrocytic merozoite form of the parasite; or

   (b) chemical or biological reproduction of the antigen.

8. A vaccine which vaccine comprises an antigen as defined in any one of claims 1 to 6 together with a pharmaceutically acceptable carrier therefor.

9. A vaccine as claimed in claim 9 suitable for inducing immunity in humans to Plasmodium falciparum.

10. An antigen, as defined in any one of claims 1 to 6, for use in inducing immunity in a susceptible vetebrate host.

11. A method for inducing immunity to malaria in a susceptible vetebrate host comprising administration to the host of an antigen, as defined in any one of claims 1 to 6.

12. An antigen as claimed in claim 10, in the form of vaccine.

13. A method as claimed in claim 11 wherein the antigen is in the form of a vaccine.

CLB/JAH/18th March, 1982.

FIG.1

FIG.2